# EUROPEAN PATENT SPECIFICATION

(11) **EP 2 405 887 B1**
(45) Date of publication and mention of the grant of the patent: **21.11.2012**
(21) Application number: 10705583.2
(22) Date of filing: 18.02.2010
(51) Int. Cl.: A61K 8/19, A61K 8/36, A61K 8/81, A61Q 19/02

(54) **A COSMETIC COMPOSITION FOR SKIN LIGHTENING**
KOSMETISCHE HAUTAUFHELLUNGSZUSAMMENSETZUNG
COMPOSITION COSMÉTIQUE POUR ÉCLAIRCIR LA PEAU

(30) Priority: 09.03.2009 IN MU05092009
(43) Date of publication of application: 18.01.2012
(73) Proprietor: Unilever NV, 3013 AL Rotterdam (NL); Unilever PLC, London Greater London EC4Y 0DY (GB)
(72) Inventor: GADGIL, Vijay Ramachandra, Bangalore 560 066 (IN); PANDEY, Parul, Bangalore 560 066 (IN); SURENDRA, Nagalakshmi, Bangalore 560 066 (IN); VELAYUDHAN NAIR, Gopa Kumar, Bangalore 560 066 (IN); VORA, Shilpa Atul, Bangalore 560 066 (IN)
(74) Representative: Acham, Nicholas Clive
(86) International application number: PCT/EP2010/052037
(87) International publication number: WO 2010/102888

(56) References cited:
- EP-A1- 1 316 306
- EP-A2- 0 345 082
- EP-A2- 0 390 456
- WO-A1-91/14759
- FR-A1- 2 897 268
- GB-A- 2 134 784

## Description

### TECHNICAL FIELD

The present invention relates to a cosmetic composition for skin lightening.

### BACKGROUND OF THE INVENTION

Consumers, particularly from Asia, prefer a lighter skin colour and therefore skin lightening has been an active area of research in the past. Some consumers believe that skin lightening agents used in the past have undesirable side effects and/or are relatively less efficacious. The skin lightening efficacy is relatively low when the consumer skin is exposed to a source of UV radiation such as sunlight.

Further, some skin lightening agents, when included in a cosmetic composition have a negative effect on the spreading characteristics and sensory feel of the composition on the skin

One of the objects of the present invention is to overcome or ameliorate at least one of the disadvantages of the prior art, or to provide a useful alternative.

Another object of the present invention is to provide a cosmetic composition for skin lightening that is relatively more efficacious.

Yet another object of the present invention is to provide a cosmetic composition for skin lightening that has relatively better spreading characteristics and acceptable sensory feel.

Present inventors have surprisingly found that certain specific salts of acetic acid viz. the potassium and magnesium salts provide enhanced efficacy in skin lightening as compared to other salts of low molecular weight carboxylic acid when incorporated in a cosmetic composition at a certain range.

EP 0 345 082 (1989) discloses a pharmaceutical agent to suppress the formation of an enzyme that prevents the formation of tyrosinase viz. members selected from the group consisting of acetic acid, lactic acid, pyruvic acid and their salts.

The disclosures in published documents heretofore do not disclose the specific selection of salts of acetic acids in a certain range for providing skin lightening.

### SUMMARY OF THE INVENTION

According to the present invention there is provided a cosmetic composition for skin lightening comprising:
(a) 0.5 to 10% by weight salt of a potassium or magnesium acetate;
(b) an organic sunscreen; and
(c) a cosmetically acceptable carrier.

### DETAILED DESCRIPTION OF THE INVENTION

The composition of the present invention is preferably for non-therapeutic use, more preferably for a cosmetic use.

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. The disclosure of the invention as found herein is to be considered to cover all embodiments as found in the claims as being multiply dependent upon each other irrespective of the fact that claims may be found without multiple dependency or redundancy.

### Potassium or magnesium acetate

The cosmetic composition comprises 0.5 to 10% by weight, preferably from 1 to 8% by weight, more preferably from 1 to 5% by weight potassium or magnesium acatete. Of these two salts, magnesium acetate is particularly preferred. The present inventors have determined that the potassium and magnesium acetates are far superior in providing skin lightening as compared to other alkali or alkali metal acetates. Further, they have also found that these two salts are superior to salts of other carboxylic acids of low molecular weight e.g. lactates, tartarates or propionates in providing skin lightening.

### Polymer

The cosmetic composition for skin lightening preferably comprises a polymer selected from a homopolymer or a copolymer of acrylic acid, vinyl alcohol, vinyl pyrrolidone, or ethylene oxide.

The polymer is preferably from 0.1 to 5% by weight, more preferably from 0.5 to 5% by weight and most preferably from 0.5 to 3% by weight of the cosmetic composition.

The polymer may be a crosslinked polymer. Preferred crosslinking agent is polyethylene glycol of divinyl benzene.

Some examples of commercially available polymers that can be used include: ACULYN 33™ (Rohm and Haas) -Acrylic acid copolymer emulsion, ROVACE™ HP-2931 (Rohm and Haas) -Vinyl acetate/acrylic copolymer, SUNSPHERES™ (Rohm and Haas)- styrene/acrylic copolymer spheres, Structure Plus™ (National starch) -Acrylates / aminoacrylates / C10-30 alkyl PEG-20 itaconate copolymer, Structure 3001™ (National starch) -Acrylates / Ceteth-20 itaconate copolymer, Structure 2001™ (National starch) -Acrylates / Steareth-20 itaconate copolymer, and Pemulen™ (Lubrizol) - Acrylates/C10-30 alkyl acrylate crosspolymer.

The molecular mass of the polymer is preferably greater than 50000, more preferably greater than 90000, and most preferably greater than 100000. The polymer, the acetate salt and an aqueous solution are preferably premixed together to form an adduct prior to adding to the composition. Without wishing to be bound by theory it is believed that the inclusion of the polymer synergistically interacts with the acetate salt in providing the right balance of spreading of the composition on the skin and the biochemical reaction necessary for the skin lightening action while not compromising on the feel and other sensory properties that the consumers expect to get from such a product.

### Cosmetically acceptable carrier

Compositions of this invention comprise a cosmetically acceptable carrier. Amount of the carrier can be up to 99.5% by weight of the composition. However, the carrier is more preferably from 70 to 95%, and most preferably from 80 to 90% by weight of the composition. Among the useful carriers are water, saturated emollients, saturated fatty acids, saturated fatty alcohols, humectants, thickeners or combinations thereof. The carrier may be aqueous, anhydrous or an emulsion.

Preferably the compositions are aqueous, especially water and oil emulsions of the W/O or O/W or triplex W/O/W variety. Water when present is preferably from 5 to 95%, more preferably from 10 to 80%, most preferably from 20 to 75% by weight of the composition.

Fatty acids having from 10 to 30 carbon atoms are also suitable as cosmetically acceptable carriers. Illustrative of this category are pelargonic, lauric, myristic, palmitic, stearic, isostearic, hydroxystearic and behenic acids. A preferred cosmetically accepted carrier is the so-called vanishing cream base. The vanishing cream base comprises fatty acid, generally stearic acid or a combination thereof with palmitic acid. Fatty acid, when present is preferably from 0.5 to 30% by weight, more preferably from 1 to 25% by weight and most preferably from 5 to 20% by weight of the cosmetic composition. The vanishing cream base also comprises salts of fatty acids, generally alkali metal soap, which acts as the emulsifier. The emulsifier is important for physical stability of the product. The soap is formed by in-situ neutralization of a portion of the fatty acid with caustic potash or any other base.

Fatty alcohols having from 10 to 30 carbon atoms are another useful category of cosmetically acceptable carrier. Illustrative of this category are stearyl alcohol, lauryl alcohol, myristyl alcohol and cetyl alcohol. Humectants of the polyhydric alcohol-type can be employed as cosmetically acceptable carriers. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1, 3-butylene glycol, isoprene glycol, 1,2, 6- hexanetriol, ethoxylated glycerol, propoxylated glycerol and mixtures thereof. The amount of humectant may range anywhere from 0.5 to 50%, preferably between 1 and 15% by weight of the composition.

The cosmetic compositions of the present invention may be in any form. These forms may include lotions, creams, roll-on formulations, sticks, mousses, aerosol and non-aerosol sprays.

Surfactants, other than soap, may also be present in personal care composition of the present invention. Total concentration of the surfactant when present may range from 0.1 to 40%, preferably from 1 to 20%, optimally from 1 to 5% by weight of the composition. The surfactant may be selected from the group consisting of anionic, nonionic, cationic and amphoteric actives. Particularly preferred nonionic surfactants are those with a C10-C20 fatty alcohol or fatty acid hydrophobe condensed with from 2 to 100 moles of ethylene oxide or propylene oxide per mole of hydrophobe; C2-C10 alkyl phenols condensed with from 2 to 20 moles of alkylen oxide; mono-and di-fatty acid esters of ethylene glycol; fatty acid monoglyceride; sorbitan, mono-and di-C8-C20 fatty acids; and polyoxyethylene sorbitan as well as combinations thereof. Alkyl polyglycosides and saccharide fatty amides (e.g. methylgluconamides) are also suitable nonionic surfactants. Preferred anionic surfactants include soap; alkyl ether sulfates and sulfonates; alkyl sulfates and sulfonates; alkylbenzene sulfonates; alkyl and dialkylsulfosuccinates; C8-C20 acyl isethionate; C8-C20 alkyl ether phosphates; C8-C20 sarcosinates; and combinations thereof.

The cosmetic composition comprises an organic sunscreen. Some examples of organic sunscreens include ethylhexyl-p-methoxycinnamate, available as Parsol MCX™; Avobenzene™, available as Parsol 1789™; and benzophenone-3, also known as Oxybenzone™.

Amounts of the sunscreen agents when present may generally range from 0.1 to 30%, preferably from 2 to 20%, optimally.from 3 to 10% by weight of the composition.

Preservatives can desirably be incorporated into the cosmetic compositions of this invention to protect against the growth of potentially harmful microorganisms. Suitable traditional preservatives for compositions of this invention are alkyl esters of parahydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from 0.01% to 2% by weight of the composition.

The cosmetic composition preferably comprises a vitamin. Illustrative vitamins are Vitamin A (retinol), Vitamin B2, Vitamin B3 (niacinamide), Vitamin B6, Vitamin C, Vitamin E and Biotin. Derivatives of the vitamins may also be employed. For instance, Vitamin C derivatives include ascorbyl tetraiso palmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed.

The total amount of vitamins, when present, in compositions according to the present invention may range from 0.001 to 10%, preferably from 0.01 % to1 %, optimally from 0.1 to 0.5% by weight of the personal care composition.

Desquamation promoters may be present. Illustrative are the alpha-hydroxycarboxylic acids and beta-hydroxycarboxylic acids. The term "acid" is meant to include not only the free acid but also salts and C1-C30 alkyl or aryl esters thereof and lactones generated from removal of water to form cyclic or linear lactone structures. Representative alpha-hydroxy acids are glycolic, lactic and malic acids. Salicylic acid is representative of the beta-hydroxycarboxylic acids. Amounts of these materials when present may range from 0.01 to 15% by weight of the composition.

Colorants, opacifiers and abrasives may also be included in compositions of the present invention. Each of these substances may range from 0.05 to 5%, preferably between 0.1 and 3% by weight of the composition.

The composition of the invention may comprise a conventional deodourant base as the cosmetically acceptable carrier. By a deodorant is meant a product in the stick, roll-on, or propellant medium which is used for personal deodorant benefit e.g. application in the under-arm or any other area which may or may not contain anti-perspirant actives. Deodorant compositions can generally be in the form of firm solids, soft solids, gels, creams, and liquids and are dispensed using applicators appropriate to the physical characteristics of the composition.

Deodorant compositions which are delivered through roll-ons generally comprise a liquid carrier. Such liquid carrier can be hydrophobic or comprise a mixture of both hydrophilic and hydrophobic liquids. They may be in the form of an emulsion or a microemulsion. The liquid carrier or mixture of carriers often constitutes from 30 to 95% and in many instances from 40 to 80% by weight of the composition.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents and skin healing agents.

The compositions of the present invention can also be, optionally, incorporated into an insoluble substrate for application to the skin such as in the form of a treated wipe.

According to yet another aspect of the present invention there is provided the cosmetic composition of the invention for use in lightening of skin.

### EXAMPLES

The invention will now be demonstrated with examples. The examples are for the purpose of illustration only and do not limit the scope of the invention in any manner.

### Test protocols

### Keratinocyte Differentiation Assay

This is an in vitro cell culture based assay to evaluate the capability of these actives to alter cell differentiation process. Keratinocytes (HaCaT cells - 5 X 10⁴ cells /well) were grown in 24 well plates for 24 hours. These proliferating keratinocytes were further cultured with actives for 24 hours. Cells were then washed with PBS and stained with acridine orange in PBS (Sigma Chemical Co, USA). Cells were washed 15 minutes later and viewed using an epi-fluorescent microscope (Olympus BX 50), at 20X magnification, using appropriate excitation and barrier filters, to simultaneously view red and green fluorescence (excitation: 460 nm and emission > 515 nm). The entire plate was scanned and the percentage of differentiated keratinocytes (cells with red fluorescence evenly distributed within the cytoplasm) was estimated. Data were represented as % of control. Higher % values indicate increased cell differentiation. The efficacy of various substances in the cell differentiation assay is tabulated below.

**Table 1: Results of cell differentiation assay**

| **Substance** | **Concentration** | **% Differentiation** |
|---|---|---|
| Sodium acetate | 0.01% | 168 |
| Potassium acetate | 0.01% | 253 |
| Magnesium acetate | 0.01% | 237 |
| Sodium acetate | 0.002% | 175 |
| Potassium acetate | 0.002% | 250 |
| Magnesium acetate | 0.002% | 250 |
| Ammonium acetate | 0.002% | 175 |
| Control | - | 100 |
| Na tartarate (c4) | 0.01% | 165 |

From the above table it is clear that potassium and magnesium acetate provide for better efficacy in keratinocyte differentiation assay as compared to similar salts of low molecular weight carboxylic acids.

### Preparation of compositions

Aqueous phase was prepared by adding potassium hydroxide, titanium dioxide, KOH, methyl paraben, and disodium EDTA. The aqueous phase was heated till 75 °C and stearic acid melted at temperature of 75 °C was added to it, followed by addition of the remaining oil phase ingredients (cetyl alcohol, isopropyl myristate, Parsol 1789, Parsol MCX and dimethicone) preheated at 75° C. The emulsion was homogenized till a milky white continuous mixture is obtained. The magnesium acetate adduct was added to the emulsion after it cooled to a temperature of about 60 °C. After the mixture is cooled to 35 °C, the other ingredients (vitamins, perfume) are added.

### Preparation of magnesium acetate adduct

1 g of magnesium acetate was dissolved in 10 g of water and to this solution 2 g of polymer (Aculyn 33) was added in a homogenizer over 5 minutes followed by slow addition of 10 mL of 10% potassium hydroxide solution to obtain magnesium acetate adduct in a viscous gel format. Magnesium acetate adduct is then added to the cream formulation after emulsification is complete. The composition of the cream after addition of magnesium acetate adduct is given below

**Table 2: Cosmetic composition**

| **Ingreient** | **% by weight** |
|---|---|
| Water | 53 |
| stearic acid | 18 |
| Cetyl alcohol | 0.5 |
| Isopropyl myristate | 0.75 |
| Parsol 1789 | 0.48 |
| Parsol MCX | 0.75 |
| Glycerine | 1.000 |
| KOH | 0.97 |
| Dimethicone(DC200) | 0.5 |
| *Magnesium Acetate ADDUCT* | |
| Aculyn 33 | 2.000 |
| KOH 10% Solution | 10.000 |
| Magnesium Acetate | 1.000 |
| Water | 10.000 |
| *Minors* (perfume, preservatives and others) | Balance |

### Effect of type of polymer on composition consistency :

The cosmetic compositions were prepared using various polymers (at the same concentration as Aculyn 33) as tabulated below along with the results in terms of formulation consistency.

**Table 3: Effect of type of polymer on composition consistency**

| **Polymer** | **Adduct form** | **Composition consistency** |
|---|---|---|
| Aculyn 33 | Gel | Thick paste |
| Polyacrylic acid (mol wt 300000) | Gel | Thick paste |
| Polyethylene oxide (mol wt 100000) | Gel | Thick paste |
| Poly vinyl alcohol (mol wt 100000) | Gel | Thick paste |
| Poly vinyl pyrrolidone (mol wt 300000) | Gel | Thick paste |
| Sodium carboxy methyl cellulose | Gel | Phase separation |
| Polyacrylic acid (mol wt 100000) | - | Phase separation |
| No polymer | - | Phase separation |

From the results it is clear that the compositions comprising magnesium acetate and specific polymers according to the present invention result into formulations with acceptable consistency without causing phase separation.

### Human Volunteer Studies

A trial was carried out for 10 days with 16 volunteers. The trial consisted of the procedure as described below:

The tests were carried out using the cosmetic composition given in Table 2 along with a control composition which was identical in all respects to the composition of Table 2 except that the control did not comprise magnesium acetate. A specific portion of the volunteer's forearm was marked out and the compositions were applied (3 mg/cm²) five times daily in about equal intervals of time. The skin lightening score was measured by expert assessors using a colour ruler on a scale of 1 to 10. The data in Table below summarizes the average skin lightening score that is the change in skin colour with respect to the initial skin colour. A more negative score indicates a higher degree of skin lightening. A more positive score indicates skin darkening. No incidence of skin irritation or erythema was reported. Spreading characteristics and sensory feel of both the compositions were acceptable to the volunteers.

**Table 4: Skin lightening efficacy in human volunteer studies**

| **Composition** | **Skin Lightening** | **% Responders** |
|---|---|---|
| Control | -0.29 | 88 |
| Composition of Table 2 | -0.35 | 94 |

From the results, it is clear that the composition according to the present invention provides a significant skin lightening (P < 0.10 significance) benefit.

It will be appreciated that cosmetic composition according to the present invention provides relatively more efficacious skin lightening and has relatively better spreading characteristics and acceptable sensory feel.

## Claims

1. A cosmetic composition for skin lightening comprising:
a. 0.5 to 10% by weight potassium or magnesium acetate;
(b) an organic sunscreen; and
(c) a cosmetically acceptable carrier.

2. A cosmetic composition for skin lightening as claimed in claim 1 comprising magnesium acetate.

3. A cosmetic composition for skin lightening as claimed in claim 1 or 2 comprising a polymer selected from a homopolymer or a copolymer of acrylic acid, vinyl alcohol, vinyl pyrrolidone, or ethylene oxide.

4. A cosmetic composition for skin lightening as claimed in any one of the preceding claims comprising a vitamin.

5. A cosmetic composition for skin lightening as claimed in any one of the preceding claims comprising from 0.5 to 30% by weight fatty acid having from 10 to 30 carbon atoms.

6. A cosmetic composition comprising:
(a) 0.5 to 10% by weight potassium or magnesium acetate; and
(b) a cosmetically acceptable carrier
for use in lightening of skin.

## Patentansprüche

1. Kosmetische Zusammensetzung zur Hautaufhellung, umfassend:
(a) 0,5 bis 10 Gew.-% Kalium- oder Magnesiumacetat;
(b) ein organisches Sonnenschutzmittel und
(c) einen kosmetisch annehmbaren Träger.

2. Kosmetische Zusammensetzung zur Hautaufhellung, wie in Anspruch 1 beansprucht, die Magnesiumacetat umfasst.

3. Kosmetische Zusammensetzung zur Hautaufhellung, wie in Anspruch 1 oder 2 beansprucht, die ein Polymer umfasst, das aus einem Homopolymer oder einem Copolymer von Acrylsäure, Vinylalkohol, Vinylpyrrolidon oder Ethylenoxid ausgewählt ist.

4. Kosmetische Zusammensetzung zur Hautaufhellung, wie in einem der vorangehenden Ansprüche beansprucht, die ein Vitamin umfasst.

5. Kosmetische Zusammensetzung zur Hautaufhellung, wie in einem der vorangehenden Ansprüche beansprucht, die 0,5 bis 30 Gew.-% Fettsäure mit 10 bis 30 Kohlenstoffatomen umfasst.

6. Kosmetische Zusammensetzung, umfassend:
(a) 0,5 bis 10 Gew.-% Kalium- oder Magnesiumacetat und
(b) einen kosmetisch annehmbaren Träger,
zur Verwendung bei der Aufhellung von Haut.

## Revendications

1. Composition cosmétique pour éclaircir la peau comprenant :
(a) de 0,5 à 10 % en poids de potassium ou d'acétate de magnésium ;
(b) un écran solaire organique ; et
(c) un vecteur acceptable sur le plan cosmétique.

2. Composition cosmétique pour éclaircir la peau selon la revendication 1 comprenant de l'acétate de magnésium.

3. Composition cosmétique pour éclaircir la peau selon la revendication 1 ou 2, comprenant un polymère choisi dans le groupe comprenant un homopolymère ou un copolymère d'acide acrylique, d'alcool vinylique, de vinylpyrrolidone ou d'oxyde d'éthylène.

4. Composition cosmétique pour éclaircir la peau selon l'une quelconque des revendications précédentes, comprenant une vitamine.

5. Composition cosmétique pour éclaircir la peau selon l'une quelconque des revendications précédentes, comprenant de 0,5 à 30 % en poids d'acide gras ayant de 10 à 30 atomes de carbone.

6. Composition cosmétique comprenant :
(a) de 0,5 à 10 % en poids de potassium ou d'acétate de magnésium ; et
(b) un vecteur acceptable sur le plan cosmétique, pour une utilisation pour éclaircir la peau.
